# EUROPÄISCHE PATENTSCHRIFT

(11) **EP 2 809 816 B1**
(45) Veröffentlichungstag und Bekanntmachung des Hinweises auf die Patenterteilung: **03.10.2018**
(21) Anmeldenummer: 13703546.5
(22) Anmeldetag: 29.01.2013
(51) Int. Cl.: C12P 21/02, C12N 9/00, C12N 9/26, C12N 9/50, C12N 9/54, C12N 15/75, C12R 1/07, C12P 1/04

(54) **EXPRESSIONSVERFAHREN**
EXPRESSION METHOD
PROCÉDÉ D'EXPRESSION

(30) Priorität: 31.01.2012 DE 102012201297
(43) Veröffentlichungstag der Anmeldung: 10.12.2014
(73) Patentinhaber: BASF SE, 67056 Ludwigshafen am Rhein (DE)
(72) Erfinder: KÜPPERS, Tobias, 4057 Basel (CH); STEFFEN, Victoria, 52428 Jülich (DE); EICHSTÄDT, Renée, Charlott, 50733 Köln (DE); EVERS, Stefan, 40822 Mettmann (DE); MAURER, Karl-Heinz, 40699 Erkrath (DE); BONGAERTS, Johannes, 41541 Dormagen (DE); HELLMUTH, Hendrik, 40237 Düsseldorf (DE); WEBER, Thomas, 41541 Dormagen (DE); O'CONNELL, Timothy, 40547 Düsseldorf (DE)
(86) Internationale Anmeldenummer: PCT/EP2013/051656
(87) Internationale Veröffentlichungsnummer: WO 2013/113689

(56) Entgegenhaltungen:
- EP-A1- 0 501 765
- EP-A1- 2 196 533
- WO-A1-91/02792
- WO-A2-99/43835
- WO-A2-02/088340
- CN-C- 100 372 937
- DE-A1-102006 032 507
- US-A1- 2011 020 938
- FENG Y. ET AL: "Fermentation of starch for enhanced alkaline protease production by constructing an alkalophilic Bacillus pumilus strain", APPLIED MICROBIOLOGY AND BIOTECHNOLOGY, Bd. 57, Nr. 1-2, 1. Oktober 2001 (2001-10-01), Seiten 153-160, XP055059544, ISSN: 0175-7598, DOI: 10.1007/s002530100765
- GIOIA JASON ET AL: "Paradoxical DNA repair and peroxide resistance gene conservation in Bacillus pumilus SAFR-032", PLOS ONE, PUBLIC LIBRARY OF SCIENCE, US, Bd. 2, Nr. 9, 1. Januar 2007 (2007-01-01), Seiten e928-1, XP002484265, ISSN: 1932-6203, DOI: 10.1371/JOURNAL.PONE.0000928 in der Anmeldung erwähnt
- SCHALLMEY MARCUS ET AL: "Developments in the use of Bacillus species for industrial production", CANADIAN JOURNAL OF MICROBIOLOGY, NRC RESEARCH PRESS, CA, Bd. 50, Nr. 1, 1. Januar 2004 (2004-01-01) , Seiten 1-17, XP009075662, ISSN: 0008-4166, DOI: 10.1139/W03-076

## Beschreibung

Die Erfindung liegt auf dem Gebiet der Biotechnologie, insbesondere der mikrobiellen Proteinsynthese. Die Erfindung betrifft insbesondere ein Verfahren zur Herstellung von Proteinen durch genetisch modifizierte Mikroorganismen und schlägt ferner Mikroorganismen vor, die in derartigen Verfahren Verwendung finden. Die Erfindung betrifft ferner Verwendungen derartiger Mikroorganismen zur Proteinherstellung.

Für die Herstellung von Wertstoffen können Mikroorganismen eingesetzt werden. Wertstoffe sind beispielsweise niedermolekulare Verbindungen, etwa Nahrungsmittelergänzungsstoffe oder pharmazeutisch wirksame Verbindungen, oder Proteine, für welche aufgrund ihrer Diversität wiederum ein großes technisches Einsatzgebiet besteht. Im ersten Fall werden die Stoffwechseleigenschaften der betreffenden Mikroorganismen zur Herstellung der Wertstoffe ausgenutzt und/oder verändert; im zweiten Fall werden vorzugsweise Mikroorganismen eingesetzt, die die Gene der interessierenden Proteine exprimieren.

Für die großtechnische, biotechnologische Produktion werden die betreffenden Mikroorganismen in Fermentern kultiviert, die den Stoffwechseleigenschaften der Mikroorganismen entsprechend ausgestaltet sind. Während der Kultivierung verstoffwechseln die Mikroorganismen das angebotene Substrat und bilden das gewünschte Produkt, das nach Beendigung der Fermentation üblicherweise von den Produktionsorganismen abgetrennt wird und aus dem Fermenterbrei und/oder dem Fermentationsmedium aufgereinigt und/oder aufkonzentriert wird. Bei der fermentativen Produktion von Proteinen werden typischerweise komplexe proteinreiche Rohstoffe als Substrat neben einer Kohlenstoffquelle (typischerweise Glukose) eingesetzt. Die Proteinproduktion entspricht damit einer Biotransformation von Substratprotein zum Zielprotein. Dies erfordert die vollständige Hydrolyse des Substratproteins in die einzelnen Aminosäuren, die dann zur Biosynthese des Zielproteins zur Verfügung stehen.

Zur Fermentation von Mikroorganismen besteht folglich ein reichhaltiger Stand der Technik, der von der Optimierung der betreffenden Stämme, beispielsweise hinsichtlich der Bildungsrate und der Nährstoffausnutzung, über die technische Gestaltung der Fermenter bis hin zur Gewinnung der Wertstoffe aus den betreffenden Mikroorganismen und/oder dem Fermentationsmedium reicht.

Der Einsatz von Bakterien in mikrobiellen Fermentationen ist grundsätzlich wünschenswert. Bakterien zeichnen sich durch kurze Generationszeiten und geringe Ansprüche an die Kultivierungsbedingungen aus. Dadurch können kostengünstige Kultivierungsverfahren bzw. Herstellungsverfahren etabliert werden. Zudem verfügt der Fachmann bei Bakterien in der Fermentationstechnik über einen reichhaltigen Erfahrungsschatz. Vorzugsweise werden grampositive Bakterien eingesetzt, da sie das herzustellende Protein (Zielprotein) in das sie umgebende Medium sezernieren.

Üblicherweise sind möglichst hohe Produktausbeuten bei der mikrobiellen Fermentation wünschenswert. Beispielsweise offenbart die internationale Patentanmeldung WO 91/02792 die verbesserte fermentative Produktion einer alkalischen Protease aus *Bacillus lentus* in einem optimierten *Bacillus licheniformis-*Stamm unter der Kontrolle genregulatorischer Sequenzen aus *Bacillus licheniformis,* insbesondere des *Bacillus licheniformis-*Promotors.

Feng Y. et al. ("Fermentation of starch for enhanced alkaline protease production by constructing an alkalophilic Bacillus pumilus strain", Applied Microbiol. Biotechnol., Bd. 57, Nr. 1-2, Seiten 153-160) offenbart einen genetisch veränderten *B. pumilus* Stamm, der Amylase exprimiert.

Zu *Bacillus licheniformis* alternative Produktionsorganismen, mit denen sich vergleichbar hohe oder sogar verbesserte Produktausbeuten erzielen lassen, sind im Stand der Technik nicht in zufrieden stellender Weise verfügbar. Es besteht weiterhin ein hoher Bedarf an mikrobiellen Fermentationsverfahren, die eine hohe Produktausbeute ermöglichen.

Der vorliegenden Erfindung liegt die Aufgabe zugrunde, eine hohe Produktausbeute, insbesondere eines Proteins, in einer mikrobiellen Fermentation zu erzielen.

Gegenstand der Erfindung ist ein Verfahren zur Herstellung eines Proteins durch einen Mikroorganismus umfassend die Verfahrensschritte
(a) Einbringen eines Expressionskonstruktes in einen Mikroorganismus, welches einen Promotor und eine für das Protein codierende Nukleinsäure umfasst;
(b) Exprimieren des Proteins in dem Mikroorganismus,
   wobei der Mikroorganismus zugehörig ist zur Art *Bacillus pumilus* und
   wobei der Mikroorganismus sporulationsgehemmt ist, vorzugsweise durch eine Veränderung des Gens spoVI (yqfD), insbesondere durch eine Deletion des Gens spolV (yqfD) oder Teilen davon.
   Ein erfindungsgemäßes Verfahren umfasst optional ferner den weiteren Verfahrensschritt
(c) Kultivieren des Mikroorganismus.

In bevorzugten erfindungsgemäßen Ausgestaltungen handelt es sich bei einem erfindungsgemäßen Verfahren folglich um ein Fermentationsverfahren.

Überraschenderweise wurde festgestellt, dass der Einsatz eines Bakteriums der Art *Bacillus pumilus* in einem derartigen Verfahren eine hohe Produktausbeute ermöglicht und daher vorteilhaft ist. Durch die Verwendung von *Bacillus pumilus* als Produktionsorganismus kann eine vorteilhafte, insbesondere gesteigerte Produktausbeute erreicht werden. Als Referenz dient diesbezüglich *Bacillus licheniformis,* ein im Stand der Technik etablierter Produktionsorganismus, der in einer Vielzahl mikrobieller Fermentationen industriell genutzt wird.

In einer bevorzugten Ausgestaltung handelt es sich bei dem erfindungsgemäßen Verfahren folglich um ein Verfahren zur Steigerung der Expression eines Proteins in einem Mikroorganismus. Eine gesteigerte Expression des Proteins liegt vor, wenn durch ein erfindungsgemäßes Verfahren eine größere Menge an Protein erhalten wird im Vergleich mit einem gleichartigen Verfahren, das sich von einem erfindungsgemäßen Verfahren lediglich dadurch unterscheidet, dass Bakterien der Art *Bacillus licheniformis,* vorzugsweise des Wildtyps, verwendet werden. Beide zu vergleichenden Verfahren werden diesbezüglich unter gleichen, möglichst optimalen Bedingungen für die Mikroorganismen und für die gleiche Dauer durchgeführt.

Ein Expressionskonstrukt ist eine Nukleinsäuresequenz, die bewirkt, dass das Protein in dem Mikroorganismus exprimiert werden kann. Es umfasst die genetische Information, also diejenige Nukleinsäuresequenz (Gen), die für das Protein codiert. Die Expression einer Nukleinsäuresequenz ist dessen Übersetzung in das bzw. die von dieser Sequenz codierte(n) Genprodukt(e), also in ein Polypeptid (Protein) bzw. in mehrere Polypeptide (Proteine). Die Begriffe Polypeptid und Protein werden in der vorliegenden Anmeldung synonym verwendet. Im Sinne der vorliegenden Erfindung bezeichnet Expression folglich die Biosynthese von Ribonukleinsäure (RNA) und Proteinen aus den genetischen Informationen. In der Regel umfasst die Expression die Transkription, also die Synthese einer Boten ("messenger")-Ribonukleinsäure (mRNA) anhand der DNA (Desoxyribonukleinsäure)-Sequenz des Gens und deren Translation in die entsprechende Polypeptidkette, die gegebenenfalls noch post-translational modifiziert werden kann. Das Exprimieren eines Proteins beschreibt folglich die Biosynthese desselben aus den genetischen Informationen, die erfindungsgemäß in dem Mikroorganismus vorliegen.

Ein Expressionskonstrukt umfasst ferner mindestens eine Nukleinsäuresequenz, vorzugsweise DNA, mit einer Steuerungsfunktion für die Expression der für das Protein bzw. die Hilfsprotease codierenden Nukleinsäuresequenz (sog. genregulatorische Sequenz). Eine genregulatorische Sequenz ist hierbei jede Nukleinsäuresequenz, deren Anwesenheit in dem Mikroorganismus die Transkriptionshäufigkeit derjenigen Nukleinsäuresequenz beeinflusst, vorzugsweise erhöht, die für das Protein codiert. Vorzugsweise handelt es sich um eine Promotor-Sequenz, da eine derartige Sequenz für die Expression einer Nukleinsäuresequenz wesentlich ist. Ein erfindungsgemäßes Expressionskonstrukt kann aber auch noch weitere genregulatorische Sequenzen umfassen, beispielsweise eine oder mehrere Enhancer-Sequenzen. Ein Expressionskonstrukt im Rahmen der Erfindung umfasst folglich mindestens eine funktionelle Einheit aus Gen und Promotor. Sie kann, muss jedoch nicht notwendigerweise, als physische Einheit vorliegen.

Das Vorhandensein von mindestens einem Promotor ist für ein erfindungsgemäßes Expressionskonstrukt wesentlich. Unter einem Promotor wird demnach eine DNA-Sequenz verstanden, die die regulierte Expression eines Gens ermöglicht. Natürlicherweise ist eine Promotorsequenz ein Bestandteil eines Gens und liegt oftmals an dessen 5'-Ende und somit vor dem RNA-kodierenden Bereich. Vorzugsweise liegt die Promotorsequenz in einem erfindungsgemäßen Expressionskonstrukt 5'-wärts von der für das Protein codierenden Nukleinsäuresequenz. Die wichtigste Eigenschaft eines Promotors ist die spezifische Wechselwirkung mit mindestens einem DNA-bindenden Protein bzw. Polypeptid, welches den Start der Transkription des Gens durch eine RNA-Polymerase vermittelt und als Transkriptionsfaktor bezeichnet wird. Häufig sind mehrere Transkriptionsfaktoren und/oder weitere Proteine am Start der Transkription durch eine RNA-Polymerase beteiligt. Ein Promotor ist demnach vorzugsweise eine DNA-Sequenz mit Promotoraktivität, d.h. eine DNA-Sequenz, an die mindestens ein Transkriptionsfaktor zur Initiation der Transkription eines Gens zumindest transient bindet. Die Stärke eines Promotors ist messbar über die Transkriptionshäufigkeit des exprimierten Gens, also über die Anzahl der pro Zeiteinheit erzeugten RNA-Moleküle, insbesondere mRNA-Moleküle. Ein Promotor eines erfindungsgemäßen Expressionskonstruktes kann ein eigener Promotor des Mikroorganismus sein. Eine derartige Promotorsequenz ist folglich natürlicherweise in dem Mikroorganismus vorhanden. Alternativ kann ein Promotor eines erfindungsgemäßen Expressionskonstruktes auch rekombinant in den Mikroorganismus eingebracht worden sein. Entsprechendes gilt auch für alle weiteren genregulatorischen Sequenzen, die ein erfindungsgemäßes Expressionskonstrukt aufweisen kann. Der Promotor in einem Expressionskonstrukt, wie es in einem erfindungsgemäßen Verfahren Verwendung findet, bewirkt die Expression der für das Protein (Zielprotein) codierenden Nukleinsäuresequenz in dem Expressionskonstrukt.

In einer bevorzugten Ausführungsform ist ein erfindungsgemäßes Verfahren dadurch gekennzeichnet, dass der Promotor eine Nukleinsäuresequenz umfasst, die ausgewählt ist aus
(a) Nukleinsäuresequenz, die zu der in SEQ ID NO: 1 angegebenen Nukleinsäuresequenz zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99%, 99,2%, 99,3%, 99,4%, 99,5% und ganz besonders bevorzugt zu 100% identisch ist;
(b) Nukleinsäuresequenz, die zu der in SEQ ID NO: 2 angegebenen Nukleinsäuresequenz zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99%, 99,2%, 99,3%, 99,4%, 99,5% und ganz besonders bevorzugt zu 100% identisch ist;
(c) Nukleinsäuresequenz, die zu der in SEQ ID NO: 3 angegebenen Nukleinsäuresequenz zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99%, 99,2%, 99,3%, 99,4%, 99,5% und ganz besonders bevorzugt zu 100% identisch ist;
(d) Nukleinsäuresequenz, die zu der in SEQ ID NO: 4 angegebenen Nukleinsäuresequenz zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99%, 99,2%, 99,3%, 99,4%, 99,5% und ganz besonders bevorzugt zu 100% identisch ist.

In einer alternativen Ausgestaltung weist der Promotor eine Nukleinsäuresequenz auf, wie sie vorstehend beschrieben ist.

Es hat sich gezeigt, dass mit derartigen Promotorsequenzen besonders hohe Produktausbeuten an Protein in einem erfindungsgemäßen Verfahren erzielt werden können.

Vorzugsweise ist der Promotor dadurch gekennzeichnet, dass er eine Nukleinsäuresequenz, die zu der in SEQ ID NO: 1 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist, umfasst, und der Promotor eine Transkriptionshäufigkeit des durch ihn exprimierten Gens bewirkt, die mindestens derjenigen eines Promotors gemäß SEQ ID NO: 1 entspricht. Alternativ ist der Promotor dadurch gekennzeichnet, dass er eine Nukleinsäuresequenz, die zu der in SEQ ID NO: 2 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist, umfasst, und der Promotor eine Transkriptionshäufigkeit des durch ihn exprimierten Gens bewirkt, die mindestens derjenigen eines Promotors gemäß SEQ ID NO: 2 entspricht.

Gemäß einer weiteren alternativen Ausführungsform ist der Promotor dadurch gekennzeichnet, dass er eine Nukleinsäuresequenz, die zu der in SEQ ID NO: 3 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist, umfasst, und der Promotor eine Transkriptionshäufigkeit des durch ihn exprimierten Gens bewirkt, die mindestens derjenigen eines Promotors gemäß SEQ ID NO: 3 entspricht. Gemäß einer anderen alternativen Ausführungsform ist der Promotor dadurch gekennzeichnet, dass er eine Nukleinsäuresequenz, die zu der in SEQ ID NO: 4 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist, umfasst, und der Promotor eine Transkriptionshäufigkeit des durch ihn exprimierten Gens bewirkt, die mindestens derjenigen eines Promotors gemäß SEQ ID NO: 4 entspricht.

In einer weiteren alternativen Ausgestaltung weist der Promotor eine Nukleinsäuresequenz auf, wie sie vorstehend beschrieben ist.

Die Bestimmung der Identität von Nukleinsäure- oder Aminosäuresequenzen erfolgt durch einen Sequenzvergleich. Solch ein Vergleich erfolgt dadurch, dass ähnliche Abfolgen in den Nukleotidsequenzen oder Aminosäuresequenzen einander zugeordnet werden. Dieser Sequenzvergleich erfolgt vorzugsweise basierend auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F.,Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- bzw. Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden üblicherweise mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen bzw. Algorithmen basieren. Im Rahmen der vorliegenden Erfindung werden Sequenzvergleiche und Alignments bevorzugt mit dem Computer-Programm Vector NTI® Suite 10.3 (Invitrogen Corporation, 1600 Faraday Avenue, Carlsbad, Kalifornien, USA) mit den vorgegebenen Standard (Default)-Parametern erstellt.

Solch ein Vergleich erlaubt eine Aussage über die Ähnlichkeit der verglichenen Sequenzen zueinander. Sie wird üblicherweise in Prozent Identität, das heißt dem Anteil der identischen Nukleotide oder Aminosäurereste an denselben bzw. in einem Alignment einander entsprechenden Positionen, angegeben. Der weiter gefasste Begriff der Homologie bezieht bei Aminosäuresequenzen konservierte Aminosäure-Austausche in die Betrachtung mit ein, also Aminosäuren mit ähnlichen Eigenschaften, da diese innerhalb des Proteins meist ähnliche Aktivitäten bzw.

Funktionen ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe bzw. identische Bereiche von verschiedenen Nukleinsäure- oder Aminosäuresequenzen sind daher durch Übereinstimmungen in den Sequenzen definiert. Sie weisen oftmals gleiche oder ähnliche Funktionen auf. Sie können klein sein und nur wenige Nukleotide bzw. Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- bzw. Homologieangaben in der vorliegenden Anmeldung aber auf die Gesamtlänge der jeweils angegebenen Nukleinsäure- oder Aminosäuresäuresequenz. Bei Proteinen, insbesondere bei Enzymen und hierunter besonders bei Proteasen, beziehen sich die Angaben ferner auf das jeweils reife (mature) Protein, soweit nicht anders angegeben. Ohne anderslautende Angaben ist eine Sequenzbetrachtung für ein Protein folglich immer auf das reife, fertig prozessierte Protein gerichtet, selbst wenn von dem zugehörigen Gen eine immature Form codiert wird, die nach der Translation noch zur reifen Form prozessiert wird.

Das in einem erfindungsgemäßen Verfahren in den Mikroorganismus einzubringende Expressionskonstrukt codiert ferner für ein Protein. Es umfasst folglich eine Nukleinsäuresequenz, die für dieses Protein codiert. Hierfür ist grundsätzlich eine beliebige Nukleinsäuresequenz verwendbar, die in ein Protein translatiert werden kann. Hierbei handelt es sich um dasjenige Protein, das mit Hilfe eines erfindungsgemäßen Verfahrens hergestellt werden soll (Zielprotein). Vorzugsweise handelt es sich um ein Enzym, weiter bevorzugt um ein Enzym wie nachstehend beschrieben.

Erfindungsgemäße Nukleinsäuren und Expressionskonstrukte können über an sich bekannte Verfahren zur Veränderung von Nukleinsäuren erzeugt werden. Solche sind beispielsweise in einschlägigen Handbüchern wie dem von Fritsch, Sambrook und Maniatis, "Molecular cloning: a laboratory manual", Cold Spring Harbour Laboratory Press, New York, 1989, dargestellt und dem Fachmann auf dem Gebiet der Biotechnologie geläufig. Beispiele für solche Verfahren sind die chemische Synthese oder die Polymerase-Kettenreaktion (PCR), optional in Verbindung mit weiteren molekularbiologischen und/oder chemischen bzw. biochemischen Standardmethoden.

Die vorliegende Erfindung eignet sich insbesondere für die rekombinante Herstellung von Proteinen, insbesondere Enzymen. Hierfür wird das Expressionskonstrukt in den Mikroorganismus eingebracht, vorzugsweise durch Transformation. Diesbezüglich erfolgt die Einschleusung des jeweiligen Expressionskonstruktes oder Teilen hiervon vorzugsweise über Vektoren, insbesondere Expressionsvektoren. Es ist aber auch möglich, dass das nur Teile des Expressionskonstruktes, vorzugsweise zumindest die Nukleinsäure, die für das Protein codiert, in den Mikroorganismus derart eingebracht werden, dass das fertige Expressionskonstrukt erst in dem Mikroorganimus entsteht. Dies kann beispielsweise durch einen Vektor erfolgen, der bewirkt, dass das Gen für das Protein in der Wirtszelle in ein bereits vorhandenes genetisches Element wie das Chromosom, die chromosomale DNA oder andere Vektoren eingefügt werden kann, so dass beispielsweise ein endogener Promotor für die Expression des Gens für das Protein genutzt wird. Der Begriff des Einbringens umfasst folglich die Möglichkeit, dass ein Expressionskonstrukt vollständig in den Mikroorganismus eingebracht, vorzugsweise transformiert, wird, aber auch die Möglichkeit, dass nur ein Teil des Expressionskonstruktes, besonders bevorzugt die Nukleinsäure, die für das Protein codiert, in den Mikroorganismus eingebracht, vorzugsweise transformiert, wird und das vollständige Expressionskonstrukt erst in dem Mikroorganismus entsteht. Zumindest ein Teil des Expressionskonstruktes wird im Rahmen der Erfindung aber immer in den Mikroorganismus eingebracht.

Vektoren sind dem Fachmann auf dem Gebiet der Biotechnologie bekannt. Sie sind insbesondere bei der Verwendung in Bakterien spezielle Plasmide, also zirkulare genetische Elemente. Im Rahmen der vorliegenden Erfindung ist das Expressionskonstrukt vorzugsweise in einen Vektor kloniert. Zu den Vektoren können beispielsweise solche gehören, die sich von bakteriellen Plasmiden, von Viren oder von Bacteriophagen ableiten, oder überwiegend synthetische Vektoren oder Plasmide mit Elementen verschiedenster Herkunft. Mit den weiteren jeweils vorhandenen genetischen Elementen vermögen Vektoren sich in den Mikroorganismen über mehrere Generationen hinweg als stabile Einheiten zu etablieren. Es ist dabei im Sinne der Erfindung unerheblich, ob sie sich extrachomosomal als eigene Einheiten etablieren oder in die chromosomale DNA integrieren. Welches der zahlreichen Systeme gewählt wird, hängt vom Einzelfall ab. Ausschlaggebend können beispielsweise die erreichbare Kopienzahl, die zur Verfügung stehenden Selektionssysteme, darunter vor allem Antibiotikaresistenzen, oder die Kultivierbarkeit der zur Aufnahme der Vektoren befähigten Mikroorganismen sein.

Expressionsvektoren können ferner durch Änderungen der Kulturbedingungen wie beispielsweise die Zelldichte oder die Zugabe von bestimmten Verbindungen regulierbar sein. Ein Beispiel für eine solche Verbindung ist das Galactose-Derivat Isopropyl-ß-D-thiogalactopyranosid (IPTG), welches als Aktivator des bakteriellen Lactose-Operons (lac-Operons) verwendet wird.

In einer weiteren Ausführungsform der Erfindung ist ein erfindungsgemäßes Verfahren dadurch gekennzeichnet, dass das Protein nicht natürlicherweise in dem Mikroorganismus vorhanden ist.

Nicht natürlicherweise vorhanden bedeutet in diesem Zusammenhang, dass das Protein kein eigenes Protein bzw. Enzym des Mikroorganismus ist. Das Protein kann folglich in dem Mikroorganismus nicht von einer Nukleinsäuresequenz exprimiert werden, die Teil der chromosomalen DNA des Mikroorganismus in seiner Wildtyp-Form ist. Das Protein und/oder die hierfür jeweils codierende Nukleinsäuresequenz ist folglich in der Wildtyp-Form des Mikroorganismus nicht vorhanden und/oder kann aus der Wildtyp-Form des Mikroorganismus nicht aus diesem isoliert werden. Vorzugsweise ist ein nicht natürlicherweise in dem Mikroorganismus vorhandenes Protein bzw. die hierfür codierende Nukleinsäuresequenz mit Hilfe gentechnischer Verfahren in den Mikroorganismus gezielt eingebracht worden, so dass der Mikroorganismus um das Protein bzw. die hierfür codierende Nukleinsäuresequenz bereichert worden ist. Jedoch kann ein Protein durchaus natürlicherweise in einem anderen Mikroorganismus vorhanden sein - relevant für die Betrachtung ist ausschließlich der in dem Verfahren eingesetzte Mikroorganismus.

In einer weiteren Ausführungsform der Erfindung ist das Verfahren dadurch gekennzeichnet, dass das Protein ein Enzym ist, insbesondere eine saure Cellulase, Alpha-Amylase, Alpha-Acetodecarboxylase, Aminopetidase, Amylase, Arabanase, Beta-Glucanase, Beta-Glucosidase, Beta-Mannosidase, Carageenase, Carbohydrase, Catalase, Cellobiose-Oxidase, Cellulase, Chymosin, Endo-1,3-Beta-Glucanase, Endo-1,3(4)-Beta-Glucanase, Endo-1,4-Beta-Xylanase, Endopeptidase, Esterase, Exopeptidase, G4-Amylase, Glucoamylase, Glucoseoxidase, Glucosidase, Glycolipase, Hemicellulase, Laccase, Lipase, Lyspohospholipase, maltogene Amylase, Mannanase, neutrale Protease, Nuklease, Oxidase, Oxidoreduktase, Pectatlyase, Pectinase, Pectinesterase, Pentosanase, Perhydrolase, Phospholipase, Phytase, Polygalacturonase, Protease, Proteinase, Pullulanase, Rennet-Enzym, Rhamnogalacturonase, Subtilisin, Tannase, Transferase, Transglutaminase, Xanthanase, Xylanase, Xyloglucanase oder Mischungen hieraus. Ganz besonders bevorzugt handelt es sich bei dem Protein um eine Protease. In besonders vorteilhaften Ausgestaltungen eines erfindungsgemäßen Verfahrens ist die zu produzierende Protease (= Zielprotease) gleichzeitig auch an der Hydrolyse des Protein-Substrats für den Mikroorganimus beteiligt und kann vorteilhafterweise einen nochmals verbesserten Aufschluss von Substratprotein bewirken. Folglich stehen dem Mikroorganismus dann verbesserte Nährbedingungen zur Verfügung.

Beispielsweise können mit einem erfindungsgemäßen Verfahren die nachstehend genannten Enzyme vorteilhaft hergestellt werden.

Unter den Proteasen sind Subtilisine bevorzugt. Beispiele hierfür sind die Subtilisine BPN' und Carlsberg, die Protease PB92, die Subtilisine 147 und 309, die Alkalische Protease aus Bacillus lentus, Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase® von der Firma Novozymes A/S, Bagsvaerd, Dänemark, erhältlich. Die Subtilisine 147 und 309 werden unter den Handelsnamen Esperase®, beziehungsweise Savinase® von der Firma Novozymes vertrieben. Von der Protease aus *Bacillus lentus* DSM 5483 leiten sich die unter der Bezeichnung BLAP® geführten Protease-Varianten ab. Weitere bevorzugte Proteasen sind ferner beispielsweise die unter der Bezeichnung PUR geführten Enzyme. Weitere Proteasen sind ferner die unter den Handelsnamen Durazym®, Relase®, Everlase®, Nafizym®, Natalase®, Kannase® und Ovozyme® von der Firma Novozymes, die unter den Handelsnamen, Purafect®, Purafect® OxP, Purafect® Prime, Excellase® und Properase® von der Firma Genencor/Danisco, das unter dem Handelsnamen Protosol® von der Firma Advanced Biochemicals Ltd., Thane, Indien, das unter dem Handelsnamen Wuxi® von der Firma Wuxi Snyder Bioproducts Ltd., China, die unter den Handelsnamen Proleather® und Protease P® von der Firma Amano Pharmaceuticals Ltd., Nagoya, Japan, und das unter der Bezeichnung Proteinase K-16 von der Firma Kao Corp., Tokyo, Japan, erhältlichen Enzyme. Bevorzugt sind ferner auch die Proteasen aus *Bacillus gibsonii* und *Bacillus pumilus,* die offenbart sind in den internationalen Patentanmeldungen WO2008/086916 und WO2007/131656.

Beispiele für Amylasen sind die α-Amylasen aus Bacillus licheniformis, aus Bacillus amyloliquefaciens oder aus Bacillus stearothermophilus sowie insbesondere auch deren für den Einsatz in Wasch- oder Reinigungsmitteln verbesserte Weiterentwicklungen. Das Enzym aus Bacillus licheniformis ist von dem Unternehmen Novozymes unter dem Namen Termamyl® und von dem Unternehmen Danisco/Genencor unter dem Namen Purastar®ST erhältlich. Weiterentwicklungsprodukte dieser α-Amylase sind von dem Unternehmen Novozymes unter den Handelsnamen Duramyl® und Termamyt®ultra, von dem Unternehmen Danisco/Genencor unter dem Namen Purastar®OxAm und von dem Unternehmen Daiwa Seiko Inc., Tokyo, Japan, als Keistase® erhältlich. Die α-Amylase von Bacillus amyloliquefaciens wird von dem Unternehmen Novozymes unter dem Namen BAN® vertrieben, und abgeleitete Varianten von der α-Amylase aus Bacillus stearothermophilus unter den Namen BSG® und Novamyl®, ebenfalls von dem Unternehmen Novozymes. Des Weiteren sind die α-Amylase aus *Bacillus sp.* A 7-7 (DSM 12368) und die Cyclodextrin-Glucanotransferase (CGTase) aus Bacillus agaradherens (DSM 9948) zu nennen. Ebenso sind Fusionsprodukte aller genannten Moleküle einsetzbar. Darüber hinaus sind die unter den Handelsnamen Fungamyl® von dem Unternehmen Novozymes erhältlichen Weiterentwicklungen der α-Amylase aus *Aspergillus niger* und *A. oryzae* geeignet. Weitere vorteilhafte Handelsprodukte sind beispielsweise die Amylase Powerase® von dem Unternehmen Danisco/Genencor und die Amylasen Amylase-LT®, Stainzyme® und Stainzyme plus®, letztere von dem Unternehmen Novozymes. Auch durch Punktmutationen erhältliche Varianten dieser Enzyme können erfindungsgemäß hergestellt werden. Weitere bevorzugte Amylasen sind offenbart in den internationalen Offenlegungsschriften WO 00/60060, WO 03/002711, WO 03/054177 und WO07/079938, auf deren Offenbarung daher ausdrücklich verwiesen wird. Erfindungsgemäß herzustellende Amylasen sind ferner vorzugsweise α-Amylasen.

Beispiele für Lipasen oder Cutinasen sind die ursprünglich aus Humicola lanuginosa (Thermomyces lanuginosus) erhältlichen, beziehungsweise weiterentwickelten Lipasen, insbesondere solche mit dem Aminosäureaustausch D96L. Sie werden beispielsweise von der Firma Novozymes unter den Handelsnamen Lipolase®, Lipolase®Ultra, LipoPrime®, Lipozyme® und Lipex® vertrieben. Des Weiteren sind beispielsweise die Cutinasen herstellbar, die ursprünglich aus *Fusarium solani pisi* und *Humicola insolens* isoliert worden sind. Von der Firma Danisco/Genencor sind beispielsweise die Lipasen beziehungsweise Cutinasen herstellbar, deren Ausgangsenzyme ursprünglich aus *Pseudomonas mendocina* und *Fusarium solanii* isoliert worden sind. Als weitere wichtige Handelsprodukte sind die ursprünglich von der Firma Gist-Brocades (inzwischen Danisco/Genencor) vertriebenen Präparationen M1 Lipase® und Lipomax® und die von der Firma Meito Sangyo KK, Japan, unter den Namen Lipase MY-30®, Lipase OF® und Lipase PL® vertriebenen Enzyme zu erwähnen, ferner das Produkt Lumafast® von der Firma Danisco/Genencor.

Beispiele für Cellulasen (Endoglucanasen, EG) umfassen Sequenzen der pilzlichen, Endoglucanase(EG)-reichen Cellulase-Präparation beziehungsweise deren Weiterentwicklungen, die von dem Unternehmen Novozymes unter dem Handelsnamen Celluzyme® angeboten wird. Die ebenfalls von dem Unternehmen Novozymes erhältlichen Produkte Endolase® und Carezyme® basieren auf der 50 kD-EG, beziehungsweise der 43 kD-EG aus Humicola insolens DSM 1800. Weitere herstellbare Handelsprodukte dieses Unternehmens sind Cellusoft®, Renozyme® und Celluclean®. Weiterhin herstellbar sind beispielsweise Cellulasen, die von dem Unternehmen AB Enzymes, Finnland, unter den Handelsnamen Ecostone® und Biotouch® erhältlich sind, und die zumindest zum Teil auf der 20 kD-EG aus Melanocarpus basieren. Weitere Cellulasen von dem Unternehmen AB Enzymes sind Econase® und Ecopulp®. Weitere geeignete Cellulasen sind aus *Bacillus sp.* CBS 670.93 und CBS 669.93, wobei die aus *Bacillus sp.* CBS 670.93 von dem Unternehmen Danisco/Genencor unter dem Handelsnamen Puradax® erhältlich ist. Weitere herstellbare Handelsprodukte des Unternehmens Danisco/Genencor sind "Genencor detergent cellulase L" und lndiAge®Neutra.

Auch durch Punktmutationen erhältliche Varianten dieser Enzyme können erfindungsgemäß hergestellt werden. Besonders bevorzugte Cellulasen sind *Thielavia terrestris* Cellulasevarianten, die in der internationalen Offenlegungsschrift WO 98/12307 offenbart sind, Cellulasen aus *Melanocarpus,* insbesondere *Melanocarpus albomyces,* die in der internationalen Offenlegungsschrift WO 97/14804 offenbart sind, Cellulasen vom EGIII-Typ aus *Trichoderma reesei,* die in der europäischen Patentanmeldung EP 1305432 offenbart sind bzw. hieraus erhältliche Varianten, insbesondere diejenigen, die offenbart sind in den europäischen Patentanmeldungen EP 1240525 und EP 1305432, sowie Cellulasen, die offenbart sind in den internationalen Offenlegungsschriften WO 1992006165, WO 96/29397 und WO 02/099091. Auf deren jeweilige Offenbarung wird daher ausdrücklich verwiesen.

Ferner können weitere Enzyme hergestellt werden, die unter dem Begriff Hemicellulasen zusammengefasst werden. Hierzu gehören beispielsweise Mannanasen, Xanthanlyasen, Xanthanasen, Pektinlyasen (=Pektinasen), Pektinesterasen, Pektatlyasen, Xyloglucanasen, Xylanasen, Pullulanasen und ß-Glucanasen. Diesbezüglich geeignete Enzyme sind beispielsweise unter den Namen Gamanase®, Pektinex AR® und Pectaway® von der Firma Novozymes, unter dem Namen Rohapec® B1L von der Firma AB Enzymes und unter dem Namen Pyrolase® von der Firma Diversa Corp., San Diego, CA, USA erhältlich. Die aus Bacillus subtilis gewonnene ß-Glucanase ist unter dem Namen Cereflo® von der Firma Novozymes erhältlich. Erfindungsgemäß besonders bevorzugte Hemicellulasen sind Mannanasen, welche beispielsweise unter den Handelsnamen Mannaway® von dem Unternehmen Novozymes oder Purabrite® von dem Unternehmen Genencor vertrieben werden.

Ferner können auch Oxidoreduktasen, beispielsweise Oxidasen, Oxygenasen, Katalasen, Peroxidasen, wie Halo-, Chloro-, Bromo-, Lignin-, Glucose- oder Manganperoxidasen, Dioxygenasen oder Laccasen (Phenoloxidasen, Polyphenoloxidasen) hergestellt werden. Als geeignete Handelsprodukte sind Denilite® 1 und 2 der Firma Novozymes zu nennen. Weitere Enzyme sind in den internationalen Patentanmeldungen WO 98/45398, WO 2005/056782, WO 2004/058961 sowie WO 2005/124012 offenbart.

In einer weiteren Ausführungsform der Erfindung ist das Verfahren dadurch gekennzeichnet, dass es sich bei dem Mikroorganismus um *Bacillus pumilus* DSM 14395 handelt. Dieser Stamm wurde am 01.03.2001 bei der DSMZ (DSMZ - Deutsche Sammlung von Mikroorganismen und Zellkulturen GmbH, Inhoffenstraße 7 B, 38124 Braunschweig, Deutschland) hinterlegt und ist von der DSMZ als ein *Bacillus pumilus* Stamm identifiziert (DSM ID 01-197). Wie die Beispiele in der vorliegenden Patentanmeldung belegen, werden mit diesem Stamm sehr gute Produktausbeuten in mikrobiellen Fermentationen erzielt.

In einer weiteren Ausführungsform der Erfindung ist der in einem erfindungsgemäßen Verfahren einzusetzende Stamm genetisch modifiziert. Durch die genetische Modifikation wird die Produktausbeute vorteilhafterweise weiter gesteigert oder eine Eigenschaft des Produktes vorteilhaft geändert. Als Produkt ist hierbei das exprimierte Protein zu verstehen, welches im Fermentationsmedium vorliegt. Beispielsweise wird dessen Geruch vermindert, dessen Farbe abgeschwächt und/oder das Produkt aufgeklärt (d.h. weniger trüb) oder dessen Dichte reduziert. Mit der genetischen Modifikation ist diesbezüglich nicht das Einbringen des Expressionskonstruktes gemäß Verfahrensschritt (a) gemeint. Vielmehr ist der in dem erfindungsgemäßen Verfahren einzusetzende Mikroorganismus der Art *Bacillus pumilus* bereits genetisch modifiziert, und zwar bevor das Expressionskonstrukt gemäß Verfahrensschritt (a) in den Mikroorganismus eingebracht wird. Vorzugsweise wird das Vorliegen der genetischen Modifikation im Vergleich mit dem *Bacillus pumilus* Wildtyp, insbesondere *Bacillus pumilus* DSM 14395, ermittelt. Als genetische Modifikation kommen diesbezüglich Substitutionen, Insertionen und/oder Deletionen in Frage. Vorteilhafterweise bewirkt die genetische Modifikation die funktionelle Änderung, beispielsweise die funktionelle Inaktivierung, eines Gens in dem Mikroorganismus. Die funktionelle Änderung, beispielsweise die funktionelle Inaktivierung, des Gens bedingt dann wiederum ein gesteigerte und/oder verbesserte Herstellung des Proteins und damit eine verbesserte Produktausbeute und/oder den Erhalt eines Produktes mit einer oder mehreren verbesserten Eigenschaften. Unter funktioneller Inaktivierung ist zu verstehen, dass das/die von diesem Gen codierte(n) Genprodukt(e) nicht mehr gebildet oder in biologisch inaktiver Form gebildet wird/werden, so dass es/sie seine Funktion(en) in dem Mikroorganismus nicht mehr ausüben kann/können. Diesbezüglich kann eine funktionelle Inaktivierung eines Gens insbesondere auch dadurch erfolgen, dass es durch ein alternatives Gen vollständig oder teilweise ersetzt wird. Dieses alternative Gen kann dann anstelle des ursprünglich vorhandenen Gens exprimiert werden. Folglich wurde das ursprünglich vorhandene Gen funktionell inaktiviert, stattdessen wird das alternative Gen exprimiert und hierdurch die funktionelle Änderung bewirkt. Das alternative Gen kann ein zum ursprünglichen Gen verwandtes Gen sein (mehr als 50% Identität zu dem ursprünglichen Gen) oder ein zum ursprünglichen Gen nicht verwandtes Gen sein (50% oder weniger Identität zu dem ursprünglichen Gen). Beispielsweise kann das alternative Gen durch Insertion in die codierende Sequenz des ursprünglichen Gens eingefügt werden. Hierdurch wird das ursprüngliche Gen funktionell inaktiviert und stattdessen das alternative Gen exprimiert. Die genetische Modifikation kann sowohl in der für das Genprodukt codierenden Sequenz als auch in einer zu dem Gen gehörenden genregulatorischen Sequenz vorliegen.

Erfindungsgemäß einzusetzende Mikroorganismen können beispielsweise hinsichtlich ihrer Anforderungen an die Kulturbedingungen verändert sein, hinsichtlich ihres Bewegungsverhaltens verändert sein, hinsichtlich ihrer Sporulationsfähigkeit verändert sein, hinsichtlich eines bestimmten Stoffwechselweges verändert sein - beispielsweise um die Bildung von Schlechtgerüchen während der Fermentation zu unterbinden -, oder auch andere oder zusätzliche Selektionsmarker aufweisen.

Diesbezüglich genetisch modifizierbar sind alle Gene in dem in einem erfindungsgemäßen Verfahren einzusetzenden *Bacillus pumilus* Stamm, zu denen eine Entsprechung in dem *Bacillus pumilus* Genom existiert, das in der Veröffentlichung von Gioia et al., PLoS ONE, 9: e928 (2007) offenbart ist. Diese Veröffentlichung beschreibt das erste vollständig sequenzierte *Bacillus pumilus* Genom. Auf diese Referenz wird ausdrücklich verwiesen.

Ferner sind alle Gene in dem in einem erfindungsgemäßen Verfahren einzusetzenden *Bacillus pumilus* Stamm genetisch modifizierbar, zu denen eine Entsprechung in einem oder mehreren der Genome der nachfolgend angegebenen Mikroorganismen existiert:
*Agrobacterium radiobacter* K84, *Agrobacterium tumefaciens* str. C58, *Agrobacterium vitis* S4,
*Arcobacter butzleri* ED-1, *Arcobacter nitrofigilis* DSM 7299, *Arcobacter sp.* L, *Aromatoleum aromaticum* EbN1, *Arthrobacter aurescens* TC1, *Arthrobacter chlorophenolicus* A6, *Arthrobacter phenanthrenivorans* Sphe3, *Arthrobacter sp.* FB24, *Bacillus amyloliquefaciens* DSM 7, *Bacillus amyloliquefaciens* FZB42, *Bacillus anthracis* str. Ames, *Bacillus atrophaeus* 1942, *Bacillus cellulosilyticus* DSM 2522, *Bacillus cereus* ATCC 10987, *Bacillus cereus* ATCC 14579, *Bacillus cereus* B4264, *Bacillus clausii* KSM-K16, *Bacillus coagulans* 36D1, *Bacillus cytotoxicus* NVH 391-98, *Bacillus halodurans* C-125, *Bacillus licheniformis* ATCC 14580, *Bacillus megaterium* DSM 319, *Bacillus pseudofirmus* OF4, *Bacillus pseudomycoides* DSM 12442 (305 parts in a CON entry),
*Bacillus pumilus* SAFR-032, *Bacillus selenitireducens* MLS10, *Bacillus subtilis* BSn5, *Bacillus subtilis* subsp. spizizenii str. W23, *Bacillus subtilis subsp. spizizenii* TU-B-10), *Bacillus subtilis subsp. subtilis* RO-NN-1, *Bacillus subtilis subsp. subtilis* str. 168, *Bacillus thuringiensis* BMB1713,
*Bacillus thuringiensis serovar konkukian* str. 97-27T, *Bacillus thuringiensis serovar thuringiensis* str. T01001, *Bacillus tusciae* DSM 2912, *Bacillus weihenstephanensis* KBAB4, *Bifidobacterium adolescentis* ATCC 15703, *Bifidobacterium animalis subsp. lactis* V9, *Bifidobacterium bifidum* PRL2010, *Bifidobacterium breve* UCC2003, *Bifidobacterium dentium* Bd1, *Bifidobacterium longum* DJO10A, *Bifidobacterium longum* NCC27051, *Bifidobacterium longum subsp. infantis* ATCC 15697, *Bradyrhizobium sp.* ORS 278, *Brevibacillus brevis* NBRC 100599, *Corynebacterium aurimucosum* ATCC 700975, *Corynebacterium diphtheriae* NCTC 13129, *Corynebacterium efficiens* YS-314, *Corynebacterium glutamicum* ATCC 13032, *Corynebacterium glutamicum* R, *Corynebacterium jeikeium* K411, *Corynebacterium kroppenstedtii* DSM 44385, *Corynebacterium pseudotuberculosis* FRC41, *Corynebacterium resistens* DSM 45100, *Corynebacterium ulcerans* BR-AD22, *Corynebacterium urealyticum* DSM 7109, *Corynebacterium variabile* DSM 44702,
*Desulfovibrio aespoeensis* Aspo-2, *Desulfovibrio alaskensis* G20, *Desulfovibrio desulfuricans subsp. desulfuricans* str. ATCC 27774, *Desulfovibrio magneticus* RS-1, *Desulfovibrio salexigens* DSM 2638, *Desulfovibrio vulgaris* RCH1, *Desulfovibrio vulgaris* str. Miyazaki F, *Desulfurobacterium thermolithotrophum* DSM 11699, *Enterobacter aerogenes* KCTC 2190, *Enterobacter asburiae* LF7a, *Enterobacter cloacae subsp. cloacae* ATCC 13047 *Enterobacter sp.* 638, *Escherichia coli* 536, *Escherichia coli* APEC O1, *Escherichia coli* CFT073, *Escherichia coli* O103:H2 str. 12009, *Escherichia coli* SE11, *Escherichia coli* SE15-, *Escherichia fergusonii* ATCC 35469T chromosome, *Ethanoligenens harbinense* YUAN-3, *Eubacterium cylindroides* T2-87 draft, *Eubacterium eligens* ATCC 27750, *Eubacterium limosum* KIST612, *Eubacterium rectale* M104/1 draft, *Eubacterium siraeum* 70/3 draft, *Exiguobacterium sibiricum* 255-15, *Exiguobacterium sp.* AT1b,
*Flavobacteriaceae bacterium* 3519-10, *Flavobacterium branchiophilum* FL-15, *Flavobacterium johnsoniae* UW101, *Flavobacterium psychrophilum* JIP02/86, *Geobacillus kaustophilus* HTA426,
*Geobacillus sp.* C56-T3, *Geobacillus sp.* WCH70, *Geobacillus sp.* Y4.1MC1, *Geobacillus sp.* Y412MC52, *Geobacillus sp.* Y412MC61, plasmid pGYMC6101, *Geobacillus thermodenitrificans* NG80-2, *Geobacillus thermoglucosidasius* C56-YS93, *Geobacter bemidjiensis* Bem Geobacter lovleyi SZ, *Geobacter metallireducens* GS-15, *Geobacter sp.* FRC-32, *Geobacter sp.* M18,
*Geobacter sp.* M21, *Geobacter sulfurreducens* PCA, *Geobacter uraniireducens* Rf4, *Gloeobacter violaceus* PCC 74212, *Gluconacetobacter diazotrophicus* PAI 5 ATCC 49037, *Gluconacetobacter xylinus* NBRC 3288, *Gluconobacter oxydans* 621H, *Hydrogenobaculum sp.* Y04AAS1,
*Lactobacillus acidophilus* 30SC, *Lactobacillus amylovorus* GRL 1112, *Lactobacillus brevis* ATCC 367, *Lactobacillus buchneri* NRRL B-30929, *Lactobacillus casei* ATCC 334, *Lactobacillus casei* BD-II, *Lactobacillus casei* BL23, *Lactobacillus casei* LC2W, *Lactobacillus crispatus* ST10,
*Lactobacillus delbrueckii subsp. bulgaricus* ND02, *Lactobacillus fermentum* CECT 5716,
*Lactobacillus gasseri* ATCC 33323, *Lactobacillus helveticus* H10, *Lactobacillus johnsonii* NCC 533,
*Lactobacillus kefiranofaciens* ZW3, *Lactobacillus plantarum* WCFS1, *Lactobacillus reuteri* SD2112,
*Lactobacillus rhamnosus* GG, *Lactobacillus rhamnosus* GG ATCC 53103, *Lactobacillus ruminis* ATCC 27782, *Lactobacillus sakei subsp. sakei* 23K, *Lactobacillus salivarius* CECT 5713),
*Lactobacillus sanfranciscensis* TMW 1.1304, *Mannheimia succiniciproducens* MBEL55E,
*Mycobacterium abscessus* chromosome, *Mycobacterium africanum* GM041182, *Mycobacterium avium* 104, *Mycobacterium bovis* BCG str. Tokyo 172, *Mycobacterium canettii* CIPT 140010059,
Mycobacterium gilvum PYR-GCK, *Mycobacterium marinum* M, *Mycobacterium smegmatis* str. MC2 155, *Mycobacterium sp.* JDM601, *Mycobacterium sp.* JLS, *Mycobacterium sp.* KMS,
*Mycobacterium sp.* MCS, *Mycobacterium sp.* Spyr1, *Mycobacterium vanbaalenii* PYR-1,
*Pseudomonas aeruginosa* NCGM2.S17, *Pseudomonas brassicacearum subsp. brassicacearum* NFM421, *Pseudomonas entomophila* L48 chromosome, *Pseudomonas fluorescens* Pf-5,
*Pseudomonas fulva* 12-X, *Pseudomonas mendocina* NK-01, *Pseudomonas putida* KT2440+,
*Pseudomonas syringae pv. phaseolicola* 1448A, *Pseudomonas stutzeri* DSM 4166, *Pseudomonas syringae pv. syringae* B728a+, *Pseudomonas syringae pv. tomato* str. DC3000/,
*Stenotrophomonas maltophilia* K279a strain K279a, *Streptobacillus moniliformis* DSM 12112,
*Streptomyces avermitilis* MA-4680, *Streptomyces bingchenggensis* BCW-1, *Streptomyces cattleya* NRRL 8057 main chromosome, *Streptomyces clavuligerus* ATCC 27064, *Streptomyces coelicolor,*
*Streptomyces flavogriseus* ATCC 33331, *Streptomyces griseus subsp. griseus* NBRC 13350,
*Streptomyces scabiei* 87.22, *Streptomyces sp.* SirexAA-E, *Streptomyces venezuelae* ATCC 10712,
*Streptomyces violaceusniger* Tu 4113, *Sulfobacillus acidophilus* TPY, *Thermobifida fusca* YX,
*Thermotoga lettingae* TMO, *Thermotoga maritima* MSB8, *Thermotoga naphthophila* RKU-10,
*Thermotoga neapolitana* DSM 4359, *Thermotoga petrophila* RKU-1, *Thermotoga sp.* RQ2,
*Thermovibrio ammonificans* HB-1, *Thermus thermophilus* HB27, *Xanthomonas albilineans,*
*Xanthomonas axonopodis pv. citrumelo* F1, *Xanthomonas axonopodis pv. citri* str. 306/,
*Xanthomonas campestris pv. campestris* str. 8004, *Xanthomonas euvesicatoria, Xanthomonas oryzae pv. oryzae*

Neben der genannten Veröffentlichung sind die modifizierbaren Gene, insbesondere die Sequenzen derselben, auch in öffentlich zugänglichen Datenbanken verfügbar, beispielsweise in der KEGG (Kyoto Encyclopedia of Genes and Genomes)-Datenbank unter http://www.genome.jp/kegg oder in den Datenbanken des NCBI (National Center for Biotechnology Information, U.S. National Library of Medicine, 8600 Rockville Pike, Bethesda, MD 20894, USA) unter http://www.ncbi.nlm.nih.gov. Die KEGG-Datenbank wurde seit 1995 von den Laboratorien um Kanehisa et al. des "Kyoto University Bioinformatics Center" und des "Human Genome Center of the University of Tokyo" entwickelt. Neben einzelnen Genen enthalten diese Datenbanken auch Informationen und Sequenzen ganzer Genome oder große Teile des Genoms unterschiedlicher Mikroorganismen.

Eine genetische Entsprechung im Sinne der vorliegenden Patentanmeldung zeichnet sich zum einen durch eine möglichst hohe Sequenzhomologie zwischen dem Gen des erfindungsgemäß einzusetzenden *Bacillus pumilus-Stammes* und dem Gen des von Gioia et al. veröffentlichten *Bacillus pumilus-Stammes* und/oder dem Gen des vorstehend angegebenen Mikroorganismus aus. Zum anderen zeichnet sich eine genetische Entsprechung durch eine gleichartige Funktion aus, d.h. die einander entsprechenden Gene des erfindungsgemäß einzusetzenden *Bacillus pumilus-*Stammes und des von Gioia et al. veröffentlichten *Bacillus pumilus-Stammes* und/oder des vorstehend angegebenen Mikroorganismus besitzen eine gleichartige Funktion in dem jeweiligen Mikroorganismus.

Mit diesen Gen- und/oder Genominformationen ist es möglich, das jeweilige Gen in dem in einem erfindungsgemäßen Verfahren einzusetzenden *Bacillus pumilus* Stamm anhand von Sequenzvergleichen zu identifizieren. Basierend auf der genetischen Information, insbesondere der Sequenzinformation, aus dem von Gioia et al. veröffentlichten Genom des *Bacillus pumilus-*Stammes und/oder aus dem Genom eines vorstehend angegebenen Mikroorganismus kann der Fachmann durch Sequenzvergleich und/oder molekularbiologische Standardmethodik die Nukleinsäuresequenz mit der höchsten Sequenzübereinstimmung in dem Genom des *Bacillus pumilus*-Stammes ermitteln, der genetisch modifiziert und dann in dem erfindungsgemäßen Verfahren eingesetzt werden soll. Die Bestätigung einer gleichartigen Funktion, d.h. einer funktionellen Entsprechung, können Vergleichsversuche mit den jeweiligen Mikroorganismen liefern, in denen jeweils das auf der Basis des Sequenzvergleichs verglichene Gen auf die gleiche Weise verändert (vorzugsweise funktionell inaktiviert) wird und beobachtet wird, ob bei beiden Mikroorganismen gleichartige Veränderungen, insbesondere phänotypische Veränderungen, auftreten. Geht beispielsweise die Veränderung, insbesondere die funktionelle Inaktivierung, des fraglichen Gens in dem von Gioia et al. veröffentlichten *Bacillus pumilus-Stamm* und/oder in dem vorstehend angegebenen Mikroorganismus mit einer Veränderung der Stoffwechselaktivität, der Bewegung oder des Sporulationsverhaltens einher und wird eine entsprechende Veränderung in dem erfindungsgemäß zu modifizierenden und einzusetzenden *Bacillus pumilus-Stamm* beobachtet, so ist hierin eine Bestätigung der korrekten Zuordnung zu sehen. Entsprechende Methoden sind Standard auf dem Gebiet der Genetik, insbesondere der Genetik von Mikroorganismen, und dem Fachmann auf diesem Gebiet umfassend bekannt.

Der erfindungsgemäße Mikroorganismus ist sporulationsgehemmt. Dies wird vorzugsweise dadurch erreicht, dass dessen Gen spolV (yqfD) bzw. dessen genetische Entsprechung funktionell inaktiviert wird, insbesondere durch eine Deletion des Gens spolV (yqfD) bzw. dessen genetischer Entsprechung oder Teilen davon. Es wurde festgestellt, dass mit einem derart sporulationsgehemmten *Bacillus pumilus-Stamm* eine besonders hohe Produktausbeute in einem erfindungsgemäßen Verfahren erreicht wird.

Die in erfindungsgemäßen Verfahren zum Einsatz kommenden Mikroorganismen können in üblicher Weise kultiviert und fermentiert werden, beispielsweise in diskontinuierlichen oder kontinuierlichen Systemen. Im ersten Fall wird ein geeignetes Nährmedium mit den Mikroorganismen beimpft und das Protein nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann.

Erfindungsgemäße Verfahren sind vorzugsweise Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Proteins. Alle Fermentationsverfahren, die auf einem erfindungsgemäßen Verfahren zur Herstellung eines Proteins beruhen, stellen Ausgestaltungen eines erfindungsgemäßen Verfahrens dar.

Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als auch in der Zellmasse bzw. Trockenmasse erreicht werden. Ferner kann die Fermentation auch so gestaltet werden, dass unerwünschte Stoffwechselprodukte herausgefiltert oder durch Zugabe von Puffer oder jeweils passende Gegenionen neutralisiert werden.

Das hergestellte Protein kann aus dem Fermentationsmedium geerntet werden. Ein solches Fermentationsverfahren ist gegenüber einer Isolation des Proteins aus dem Mikroorganismus, d.h. einer Produktaufbereitung aus der Zellmasse (Trockenmasse) bevorzugt. Dies kann beispielsweise erreicht werden durch die Zurverfügungstellung von geeigneten Sekretionsmarkern bzw. -mechanismen und/oder Transportsystemen, damit die Mikroorganismen das Protein in das Fermentationsmedium sezernieren. Ohne Sekretion kann alternativ die Isolation des Proteins aus der Wirtszelle, d.h. eine Aufreinigung desselben aus der Zellmasse, erfolgen, beispielsweise durch Fällung mit Ammoniumsulfat oder Ethanol, oder durch chromatographische Reinigung.

Ein weiterer Gegenstand der Erfindung ist ein Mikroorganismus, der durch ein Verfahren erhältlich ist, welches die folgenden Verfahrensschritte umfasst:
(a) Einbringen eines Expressionskonstruktes in einen Mikroorganismus, welches einen Promotor und eine für das Protein codierende Nukleinsäure umfasst;
(b) Exprimieren des Proteins in dem Mikroorganismus,
wobei der Mikroorganismus zugehörig ist zur Art *Bacillus pumilus.*

Hierbei handelt es sich demnach um alle Mikroorganismen, die Gegenstand eines erfindungsgemäßen Verfahrens sein können. Alle Sachverhalte, Gegenstände und Ausführungsformen, die für erfindungsgemäße Verfahren beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die erfindungsgemäßen Mikroorganismen gilt.

Besonders bevorzugte Ausführungsformen erfindungsgemäßer Mikroorganismen sind dadurch gekennzeichnet, dass
(a) der Promotor eine Nukleinsäuresequenz umfasst, die ausgewählt ist aus
   (i) Nukleinsäuresequenz, die zu der in SEQ ID NO: 1 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist;
   (ii) Nukleinsäuresequenz, die zu der in SEQ ID NO: 2 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist,
   (iii) Nukleinsäuresequenz, die zu der in SEQ ID NO: 3 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist;
   (iv) Nukleinsäuresequenz, die zu der in SEQ ID NO: 4 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist,
      und/oder
(b) das Protein nicht natürlicherweise in dem Mikroorganismus vorhanden ist, und/oder
(c) das Protein ein Enzym ist, insbesondere eine saure Cellulase, Alpha-Amylase, Alpha-Acetodecarboxylase, Aminopetidase, Amylase, Arabanase, Beta-Glucanase, Beta-Glucosidase, Beta-Mannosidase, Carageenase, Carbohydrase, Catalase, Cellobiose-Oxidase, Cellulase, Chymosin, Endo-1,3-Beta-Glucanase, Endo-1,3(4)-Beta-Glucanase, Endo-1,4-Beta-Xylanase, Endopeptidase, Esterase, Exopeptidase, G4-Amylase, Glucoamylase, Glucoseoxidase, Glucosidase, Glycolipase, Hemicellulase, Laccase, Lipase, Lyspohospholipase, maltogene Amylase, Mannanase, neutrale Protease, Nuklease, Oxidase, Oxidoreduktase, Pectatlyase, Pectinase, Pectinesterase, Pentosanase, Perhydrolase, Phospholipase, Phytase, Polygalacturonase, Protease, Proteinase, Pullulanase, Rennet-Enzym, Rhamnogalacturonase, Subtilisin, Tannase, Transferase, Transglutaminase, Xanthanase, Xylanase, Xyloglucanase, bevorzugt Protease oder Alpha-Amylase, oder Mischungen hieraus, und/oder
(d) es sich bei dem Mikroorganismus um *Bacillus pumilus* DSM 14395 handelt, und/oder
(e) der Mikroorganismus sporulationsgehemmt ist, vorzugsweise durch eine Veränderung des Gens spolV (yqfD), insbesondere durch eine Deletion des Gens spolV (yqfD) oder Teilen davon, und/oder
(f) der Mikroorganismus genetisch modifiziert ist.

Eine ganz besonders bevorzugte Ausführungsformen erfindungsgemäßer Mikroorganismen sind dadurch gekennzeichnet, dass
(a) der Promotor eine Nukleinsäuresequenz umfasst, die ausgewählt ist aus
   (i) Nukleinsäuresequenz, die zu der in SEQ ID NO: 1 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist;
   (ii) Nukleinsäuresequenz, die zu der in SEQ ID NO: 2 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist,
      und/oder
(b) das Protein nicht natürlicherweise in dem Mikroorganismus vorhanden ist, und/oder
(c) das Protein eine Protease, bevorzugt ein Subtilisin, oder eine Alpha-Amylase ist und/oder
(d) es sich bei dem Mikroorganismus um *Bacillus pumilus* DSM 14395 handelt, und/oder
(e) der Mikroorganismus sporulationsgehemmt ist, vorzugsweise durch eine Veränderung des Gens spolV (yqfD), insbesondere durch eine Deletion des Gens spolV (yqfD) oder Teilen davon, und/oder
(f) der Mikroorganismus genetisch modifiziert ist.

Eine weitere ganz besonders bevorzugte Ausführungsformen erfindungsgemäßer Mikroorganismen sind dadurch gekennzeichnet, dass
(a) der Promotor eine Nukleinsäuresequenz umfasst, die ausgewählt ist aus
   (i) Nukleinsäuresequenz, die zu der in SEQ ID NO: 1 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist;
   (ii) Nukleinsäuresequenz, die zu der in SEQ ID NO: 2 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist,
   (iii) Nukleinsäuresequenz, die zu der in SEQ ID NO: 3 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist;
   (iv) Nukleinsäuresequenz, die zu der in SEQ ID NO: 4 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist,
      und/oder
(b) das Protein nicht natürlicherweise in dem Mikroorganismus vorhanden ist, und/oder
(c) das Protein eine Alpha-Amylase ist
   und/oder
(d) es sich bei dem Mikroorganismus um *Bacillus pumilus* DSM 14395 handelt, und/oder
(e) der Mikroorganismus sporulationsgehemmt ist, vorzugsweise durch eine Veränderung des Gens spolV (yqfD), insbesondere durch eine Deletion des Gens spolV (yqfD) oder Teilen davon, und/oder
(f) der Mikroorganismus genetisch modifiziert ist.

Eine in höchstem Maße besonders bevorzugte Ausführungsformen erfindungsgemäßer Mikroorganismen sind dadurch gekennzeichnet, dass
(a) der Promotor eine Nukleinsäuresequenz umfasst, die zu der in SEQ ID NO: 3 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist;
   und/oder
(b) das Protein nicht natürlicherweise in dem Mikroorganismus vorhanden ist, und/oder
(c) das Protein eine Alpha-Amylase ist
   und/oder
(d) es sich bei dem Mikroorganismus um *Bacillus pumilus* DSM 14395 handelt, und/oder
(e) der Mikroorganismus sporulationsgehemmt ist, vorzugsweise durch eine Veränderung des Gens spolV (yqfD), insbesondere durch eine Deletion des Gens spolV (yqfD) oder Teilen davon, und/oder
(f) der Mikroorganismus genetisch modifiziert ist.

Eine weitere in höchstem Maße besonders bevorzugte Ausführungsformen erfindungsgemäßer Mikroorganismen sind dadurch gekennzeichnet, dass
(a) der Promotor eine Nukleinsäuresequenz umfasst, die zu der in SEQ ID NO: 1 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist;
   und/oder
(b) das Protein nicht natürlicherweise in dem Mikroorganismus vorhanden ist, und/oder
(c) das Protein eine Alpha-Amylase ist
   und/oder
(d) es sich bei dem Mikroorganismus um *Bacillus pumilus* DSM 14395 handelt, und/oder
(e) der Mikroorganismus sporulationsgehemmt ist, vorzugsweise durch eine Veränderung des Gens spolV (yqfD), insbesondere durch eine Deletion des Gens spolV (yqfD) oder Teilen davon, und/oder
(f) der Mikroorganismus genetisch modifiziert ist.

Eine in höchstem Maße besonders bevorzugte Ausführungsformen erfindungsgemäßer Mikroorganismen sind dadurch gekennzeichnet, dass
(a) der Promotor eine Nukleinsäuresequenz umfasst, die zu der in SEQ ID NO: 2 angegebenen Nukleinsäuresequenz zu mindestens 80% und zunehmend bevorzugt zu mindestens 81,0%, 82,0%, 83,0%, 84,0%, 85,0%, 86,0%, 87,0%, 88,0%, 89,0%, 90,0%, 91,0%, 92,0%, 93,0%, 94,0%, 95,0%, 96,0%, 97,0%, 98,0%, 99,0%, 99,2%, 99,3%, 99,4%, 99,5% und ganz besonders bevorzugt zu 100% identisch ist;
   und/oder
(b) das Protein nicht natürlicherweise in dem Mikroorganismus vorhanden ist, und/oder
(c) das Protein eine Alpha-Amylase ist
   und/oder
(d) es sich bei dem Mikroorganismus um *Bacillus pumilus* DSM 14395 handelt, und/oder
(e) der Mikroorganismus sporulationsgehemmt ist, vorzugsweise durch eine Veränderung des Gens spolV (yqfD), insbesondere durch eine Deletion des Gens spolV (yqfD) oder Teilen davon, und/oder
(f) der Mikroorganismus genetisch modifiziert ist.

Erfindungsgemäße Mikroorganismen werden vorteilhaft in erfindungsgemäßen Verfahren verwendet, um ein Protein herzustellen. Konsequenterweise ist demnach ein weiterer Gegenstand der Erfindung die Verwendung eines erfindungsgemäßen Mikroorganismus zur Herstellung eines Proteins, insbesondere eines Enzyms.

Alle Sachverhalte, Gegenstände und Ausführungsformen, die für erfindungsgemäße Verfahren bzw. Mikroorganismen beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die erfindungsgemäßen Verwendungen gilt.

### Beispiele

Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder vergleichbaren einschlägigen Werken angegeben sind. Enzyme, Baukästen (Kits) und Geräte wurden nach den Angaben der jeweiligen Hersteller eingesetzt.

### Beispiel 1:

Vergleich der fermentativen Produktion einer Protease (Zielprotein) mit *Bacillus pumilus* und *Bacillus licheniformis*

Drei unterschiedliche Expressionsplasmide wie nachstehend angegeben, die jeweils ein Gen codierend für eine Protease (Zielprotein) sowie einen funktionellen Promotor umfassen, wurden sowohl in einen *Bacillus licheniformis-* als auch in einen *Bacillus pumilus-Stamm* transformiert. Die transformierten Stämme wurden für die fermentative Proteaseproduktion verwendet. Der verwendete *Bacillus licheniformis*-Stamm ist offenbart in der internationalen Patentanmeldung WO 91/02792. Als *Bacillus pumilus-Stamm* wurde *Bacillus pumilus* DSM 14395 verwendet, in dem das Gen spolV (yqfD) durch eine Deletion funktionell inaktiviert wurde. Als Promotoren dienten Nukleinsäuresequenzen gemäß SEQ ID NO: 1 und SEQ ID NO: 2. Der Promotor ist in den jeweiligen Expressionsplasmiden jeweils 5'-wärts der Nukleinsäuresequenz angeordnet, die für die Protease codiert. Folgende Plasmide wurden verwendet (Tabelle 1):

**Tabelle 1:**

| Plasmid Nr. | Promotor | Protease-Gen |
|---|---|---|
| 1 | SEQ ID NO: 1 | codierend für die Variante F49 gemäß WO 95/23221 |
| 2 | SEQ ID NO: 1 | codierend für die Variante F49 gemäß WO 95/23221 |
| 3 | SEQ ID NO: 2 | codierend für die Variante F49 gemäß WO 95/23221 |

Nach der Transformation der Expressionsplasmide in die jeweiligen Mikroorganismen wurden die resultierenden Produktionsstämme in einem Standardfermentationsverfahren in einem 2 Liter-Laborfermenter (48h Dauer der Kultur) eingesetzt und die erhaltenen Proteaseaktivitäten bestimmt über die Freisetzung des Chromophors para-Nitroanilin (pNA) aus dem Substrat suc-L-Ala-L-Ala-L-Pro-L-Phe-p-Nitroanilid (AAPF). Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6, und einer Wellenlänge von 410 nm. Die Messzeit beträgt 5 min und das Messintervall 20s bis 60s.

Verglichen mit *Bacillus licheniformis* stieg die Ausbeute mit *Bacillus pumilus* als Produktionsorganismus deutlich an (vgl. Tabelle 2). Angegeben sind die relativen gemessenen Proteaseaktivitäten für *Bacillus pumilus,* die auf die jeweils erhaltene Proteaseaktivität für *Bacillus licheniformis,* die als 100% definiert wurde, bezogen sind.

**Tabelle 2:**

| Plasmid Nr. | relative Proteaseaktivität (%) |
|---|---|
| 1 | 114 |
| 2 | 134 |
| 3 | 150 |

### Beispiel 2:

In diesem Beispiel wurde die fermentative Produktion einer Protease (Zielprotein) in *Bacillus pumilus* mit Expressionskonstrukten, die unterschiedliche Promotoren umfassten, untersucht. Verwendet wurden die Expressionsplasmide 1 und 3 mit Promotoren gemäß SEQ ID NO. 1 und SEQ ID NO. 2 wie in Beispiel 1 beschrieben. Als weiteres Expressionsplasmid (Kontrolle) wurde ein Expressionsplasmid verwendet, das sich von den Plasmiden 1 und 3 dadurch unterscheidet, dass anstelle eines Promotors aus *Bacillus pumilus* ein *Bacillus licheniformis* Promotor verwendet wurde, der in der internationalen Patentanmeldung WO 91/02792 offenbart ist ("promotor of the ATCC 53926 alkaline protease gene"; vgl. Beispiele 5, 6 und Figur 27 WO 91/02792). Als *Bacillus pumilus-*Stamm wurde wie in Beispiel 1 *Bacillus pumilus* DSM 14395 verwendet, in dem das Gen spolV (yqfD) durch eine Deletion funktionell inaktiviert wurde.

Dieser Stamm wurde mit den genannten Expressionsplasmiden transformiert. Die resultierenden Produktionsstämme wurden in einem Standardfermentationsverfahren in einem 2 Liter-Laborfermenter eingesetzt und die erhaltenen Proteaseaktivitäten bestimmt über die Freisetzung des Chromophors para-Nitroanilin (pNA) aus dem Substrat suc-L-Ala-L-Ala-L-Pro-L-Phe-p-Nitroanilid (AAPF) wie in Beispiel 1 beschrieben. Verglichen mit dem Kontrollstamm stieg die Ausbeute mit den Plasmiden 1 und 3 deutlich an (vgl. Tabelle 3). Angegeben sind die relativen gemessenen Proteaseaktivitäten für die Stämme enthaltend die Plasmide 1 und 3, die auf die Proteaseaktivität für den Kontrollstamm, die als 100% definiert wurde, bezogen sind.

**Tabelle 3:**

| Plasmid Nr. | relative Proteaseaktivität (%) |
|---|---|
| Kontrolle | 100 |
| 1 | 133 |
| 3 | 131 |

### Beispiel 3:

Zwei unterschiedliche Expressionsplasmide, wie nachstehend angegeben, die jeweils ein Gen codierend für eine Amylase (Zielprotein) sowie einen funktionellen Promotor umfassen, wurden sowohl in einen *Bacillus licheniformis-* als auch in einen *Bacillus pumilus-Stamm* transformiert. Die transformierten Stämme wurden für die fermentative Amylaseproduktion verwendet. Der verwendete *Bacillus licheniformis-*Stamm ist offenbart in der internationalen Patentanmeldung WO 91/02792. Als *Bacillus pumilus-Stamm* wurde *Bacillus pumilus* DSM 14395 verwendet, in dem das Gen spolV (yqfD) durch eine Deletion funktionell inaktiviert wurde. Als Promotoren dienten Nukleinsäuresequenzen gemäß SEQ ID NO: 3 und SEQ ID NO: 4 (Amylase Promotor aus Bacillus amyloliquefaciens, wie in Palva,l., Pettersson,R.F., Kalkkinen,N., Lehtovaara,P., Sarvas,M., Soderlund,H., Takkinen,K. and Kaariainen,L. "Nucleotide sequence of the promoter and NH2-terminal signal peptide region of the alpha-amylase gene from Bacillus amyloliquefaciens"; Gene 15 (1), 43-51 (1981) offenbart). Der Promotor ist in den jeweiligen Expressionsplasmiden jeweils 5'-wärts der Nukleinsäuresequenz angeordnet, die für die Amylase codiert. Folgende Plasmide wurden verwendet (Tabelle 4):

**Tabelle 4:**

| Plasmid Nr. | Promotor | Amylase-Gen |
|---|---|---|
| 4 | SEQ ID NO. 3 | Codierend für das Protein nach Seq ID No: 2 aus EP1307547 A2 |
| 5 | SEQ ID NO. 4 | Codierend für das Protein nach Seq ID No: 2 aus EP1307547 A2 |
| 6 | SEQ ID NO. 1 | Codierend für das Protein nach Seq ID No: 2 aus EP1307547 A2 |
| 7 | SEQ ID NO. 2 | Codierend für das Protein nach Seq ID No: 2 aus EP1307547 A2 |

Nach der Transformation der Expressionsplasmide in die jeweiligen Mikroorganismen wurden die resultierenden Produktionsstämme in einem Standardfermentationsverfahren in einem 2 Liter-Laborfermenter (48h Dauer der Kultur) eingesetzt und die erhaltenen Amylaseaktivitäten bestimmt Zur Bestimmung der amylolytischen Aktivität in TAU wird ein modifiziertes p-Nitrophenylmaltoheptaosid verwendet, dessen endständige Glucoseeinheit durch eine Benzylidengruppe blockiert ist; dieses wird durch Amylase zu freiem p-Nitrophenyl-Oligosaccharid gespalten, welches seinerseits mittels der Hilfesenzyme Glucoamylase und alpha-Glucosidase zu Glucose und p-Nitrophenol umgesetzt wird. Damit ist die Menge an freigesetztem p-Nitrophenol der Amylase-Aktivität proportional. Die Messung erfolgt beispielsweise mit dem Quick-Start®-Testkit der Fa. Abbott, Abott Park, Illinois, USA. Die Absorptionszunahme (405 nm) im Testansatz wird bei 37°C über 3 min gegen einen Blank-Wert mittels Photometer detektiert. Die Kalibrierung erfolgt über einen Enzymstandard von bekannter Aktivität (zum Beispiel Maxamyl®/Purastar® 2900 der Firma Genencor mit 2900 TAU/g). Die Auswertung erfolgt mittels Auftragung der Absorptionsdifferenz dE (405 nm) pro min gegen die Enzymkonzentration des Standards.

In Tabelle 5 sind die die relativen gemessenen Amylaseaktivitäten für *Bacillus pumilus* angegeben, die auf die mit Plasmid 4 (Promotor nach SEQ ID NO: 3) erhaltene Amylaseaktivität für *Bacillus licheniformis,* die als 100% definiert wurde, bezogen sind.

**Tabelle 5:**

| Plasmid Nr. | relative Amylaseaktivität (%) in *B. licheniformis* | relative Amylaseaktivität (%) in *B. pumilus* |
|---|---|---|
| 4 | 100 % | 376 % |
| 5 | Nicht bestimmt | 212 % |

Überraschenderweise wurde gefunden, dass der Promotor nach SEQ ID NO: 3 in *B. pumilus* (der natürlicherweise keine eigene Amylase produziert) besonders geeignet ist, eine sehr hohe Ausbeute an heterolog exprimierter Amylase zu erzielen.

### Beispiel 4:

In diesem Beispiel wurde die fermentative Produktion einer Amylase (Zielprotein) in *Bacillus pumilus* mit Expressionskonstrukten, die unterschiedliche Promotoren umfassten, untersucht. Verwendet wurden die Expressionsplasmide 4, 6 und 7 mit Promotoren gemäß SEQ ID NO. 3, 1 und SEQ ID NO: 2 wie in Beispiel 3 beschrieben. Als *Bacillus pumilus-Stamm* wurde wie in Beispiel 1 *Bacillus pumilus* DSM 14395 verwendet, in dem das Gen spolV (yqfD) durch eine Deletion funktionell inaktiviert wurde. Dieser Stamm wurde mit den genannten Expressionsplasmiden transformiert. Die resultierenden Produktionsstämme wurden in einem Standardfermentationsverfahren in einem 2 Liter-Laborfermenter eingesetzt und die erhaltenen Amylaseaktivitäten, wie in Beispiel 3 beschrieben, bestimmt.

In Tabelle 6 sind die relativen gemessenen Amylaseaktivitäten angegeben für die o.g. *B. pumilus* Stämme, enthaltend die Plasmide 4, 6 und 7, die auf die Amylaseaktivität für den *B. pumilus* Stamm, enthaltend Plasmid 4, die als 100% definiert wurde, bezogen sind.

Verglichen mit dem bereits für die heterologe Amylaseexpression in *B. pumilus* besonders geeigneten Plasmid 4 (vgl. Tabelle 5) konnte mit dem Plasmid 7 eine ähnlich hohe Ausbeute und mit dem Plasmid 6 sogar noch eine deutlich verbesserte Amylaseausbeute erzielt werden (vgl. Tabelle 6).

**Tabelle 6:**

| Plasmid Nr. | relative Amylaseaktivität (%) |
|---|---|
| 4 | 100 |
| 6 | 112 |
| 7 | 101 |

## Patentansprüche

1. Verfahren zur Herstellung eines Proteins durch einen Mikroorganismus umfassend dieVerfahrensschritte
(a) Einbringen eines Expressionskonstruktes in einen Mikroorganismus, welches einen Promotor und eine für das Protein codierende Nukleinsäure umfasst;
(b) Exprimieren des Proteins in dem Mikroorganismus,
wobei der Mikroorganismus zugehörig ist zur Art *Bacillus pumilus* und wobei der Mikroorganismus sporulationsgehemmt ist, vorzugsweise durch eine Veränderung des Gens spolV (yqfD), insbesondere durch eine Deletion des Gens spolV (yqfD) oder Teilen davon.

2. Verfahren nach Anspruch 1, **dadurch gekennzeichnet, dass** das Protein sezerniert wird.

3. Verfahren nach Anspruch 1 oder2, **dadurch gekennzeichnet, dass** der Promotor eine Nukleinsäuresequenz umfasst, die ausgewählt ist aus
(a) Nukleinsäuresequenz, die zu der in SEQ ID NO. 1 angegebenen Nukleinsäuresequenz zu mindestens 80% identisch ist;
(b) Nukleinsäuresequenz, die zu der in SEQ ID NO. 2 angegebenen Nukleinsäuresequenz zu mindestens 80% identisch ist;
(c) Nukleinsäuresequenz, die zu der in SEQ ID NO. 3 angegebenen Nukleinsäuresequenz zu mindestens 80% identisch ist;
(d) Nukleinsäuresequenz, die zu der in SEQ ID NO. 4 angegebenen Nukleinsäuresequenz zu mindestens 80% identisch ist.

4. Verfahren nach einem der Anspruch 1 bis 3, **dadurch gekennzeichnet, dass** das Protein nicht natürlicherweise in dem Mikroorganismus vorhanden ist.

5. Verfahren nach einem der Ansprüche 1 bis 4, **dadurch gekennzeichnet, dass** das Protein ein Enzym ist, insbesondere eine saure Cellulase, Alpha-Amylase, Alpha-Acetodecarboxylase, Aminopetidase, Amylase, Arabanase, Beta- Glucanase, Beta-Glucosidase, Beta-Mannosidase, Carrageenase, Carbohydrase, Catalase, Cellobiose-Oxidase, Cellulase, Chymosin, Endo-1,3-Beta-Glucanase, Endo-1,3(4)-Beta-Glucanase, Endo-1,4-Beta-Xylanase, Endopeptidase, Esterase, Exopeptidase, G4-Amylase, Glucoamylase, Glucoseoxidase, Glucosidase, Glycolipase, Hemicellulase, Laccase, Lipase, Lyspohospholipase, maltogene Amylase, Mannanase, neutrale Protease, Nuklease, Oxidase, Oxidoreduktase, Pectatlyase, Pectinase, Pectinesterase, Pentosanase, Perhydrolase, Phospholipase, Phytase, Polygalacturonase, Protease, Proteinase, Pullulanase, Rennet-Enzym, Rhamnogalacturonase, Subtilisin, Tannase, Transferase, Transglutaminase, Xanthanase, Xylanase, Xyloglucanase oder Mischungen hieraus, besonders bevorzugt Alpha-Amylase und/oder Protease.

6. Verfahren nach einem der Ansprüche 1 bis 5, **dadurch gekennzeichnet, dass** es sich bei dem Mikroorganismus um *Bacillus pumilus* DSM 14395 handelt.

7. Verfahren nach einem der Ansprüche 1 bis 6, **dadurch gekennzeichnet, dass** der Mikroorganismusgenetisch modifiziert ist.

8. Mikroorganismus enthaltend ein Expressionskonstrukt, welches einen Promotor und eine für das Protein codierende Nukleinsäure umfasst;
wobei der Mikroorganismus zugehörig ist zur Art *Bacillus pumilus* und
wobei der Mikroorganismus sporulationsgehemmt ist, vorzugsweise durch eine Veränderung des Gens spolV (yqfD), insbesondere durch eine Deletion des Gens spolV (yqfD) oder Teilen davon und
wobei der Mikroorganismus das Protein exprimiert.

9. Mikroorganismus nach Anspruch 8, **dadurch gekennzeichnet, dass** das Protein sezerniertwird.

10. Mikroorganismus nach Anspruch 8 oder 9, **dadurch gekennzeichnet, dass**
(a) der Promotor eine Nukleinsäuresequenz umfasst, die ausgewählt ist aus
(i) Nukleinsäuresequenz, die zu der in SEQ ID NO. 1 angegebenen Nukleinsäuresequenz zu mindestens 80% identisch ist;
(ii) Nukleinsäuresequenz, die zu der in SEQ ID NO. 2 angegebenen Nukleinsäuresequenz zu mindestens 80% identisch ist,
(iii) Nukleinsäuresequenz, die zu der in SEQ ID NO. 3 angegebenen Nukleinsäuresequenz zu mindestens 80% identisch ist;
(iv) Nukleinsäuresequenz, die zu der in SEQ ID NO. 4 angegebenen Nukleinsäuresequenz zu mindestens 80% identisch ist;
und/oder
(b) das Protein nicht natürlicherweise in dem Mikroorganismus vorhanden ist, und/oder
(c) das Protein ein Enzym ist, insbesondere eine saure Cellulase, Alpha-Amylase, Alpha-Acetodecarboxylase, Aminopetidase, Amylase, Arabanase, Beta-Glucanase, Beta-Glucosidase, Beta-Mannosidase, Carrageenase, Carbohydrase, Catalase, Cellobiose-Oxidase, Cellulase, Chymosin, Endo-1,3-Beta-Glucanase, Endo-1,3(4)-Beta-Glucanase, Endo-1,4-Beta-Xylanase, Endopeptidase, Esterase, Exopeptidase,.G4-Amylase, Glucoamylase, Glucoseoxidase, Glucosidase, Glycolipase, Hemicellulase, Laccase, Lipase, Lyspohospholipase, maltogene Amylase, Mannanase, neutrale Protease, Nuklease, Oxidase, Oxidoreduktase, Pectatlyase, Pectinase, Pectinesterase, Pentosanase, Perhydrolase, Phospholipase, Phytase, Polygalacturonase, Protease, Proteinase, Pullulanase, Rennet-Enzym, Rhamnogalacturonase, Subtilisin, Tannase, Transferase, Transglutaminase, Xanthanase, Xylanase, Xyloglucanase, insebesondere Protease oder Alpha-Amylase, oder Mischungen hieraus
und/oder
(d) es sich bei dem Mikroorganismus um *Bacillus pumilus* DSM 14395 handelt, und/oder
(e) der Mikroorganismus genetisch modifiziert ist.

11. Verwendung eines Mikroorganismus nach einem der Ansprüche 8 bis 10 zur Herstellung eines Proteins, insbesondere eines Enzyms.

## Claims

1. A method for producing a protein by means of a microorganism, comprising the method steps of
(a) introducing an expression construct into a microorganism, which expression construct comprises a promoter and a nucleic acid coding for the protein;
(b) expressing the protein in the microorganism, the microorganism belonging to the species *Bacillus pumilus* and
the microorganism being sporulation-inhibited, preferably by means of a modification of the gene spoIV (yqfD), more particularly by means of a deletion of the gene spoIV (yqfD) or parts thereof.

2. The method according to claim 1, wherein the protein is secreted.

3. The method according to claim 1 or 2, wherein the promoter comprises a nucleic acid sequence selected from
(a) nucleic acid sequence which is at least 80% identical to the nucleic acid sequence specified in SEQ ID NO. 1;
(b) nucleic acid sequence which is at least 80% identical to the nucleic acid sequence specified in SEQ ID NO. 2;
(c) nucleic acid sequence which is at least 80% identical to the nucleic acid sequence specified in SEQ ID NO. 3;
(d) nucleic acid sequence which is at least 80% identical to the nucleic acid sequence specified in SEQ ID NO. 4.

4. The method according to any of claims 1 to 3, wherein the protein is not naturally present in the microorganism.

5. The method according to any of claims 1 to 4, wherein the protein is an enzyme, more particularly an acidic cellulase, alpha-amylase, alpha-acetodecarboxylase, aminopeptidase, amylase, arabanase, beta-glucanase, beta-glucosidase, beta-mannosidase, carrageenase, carbohydrase, catalase, cellobiose oxidase, cellulase, chymosin, endo-1,3-beta-glucanase, endo-1,3(4)-beta-glucanase, endo-1,4-beta-xylanase, endopeptidase, esterase, exopeptidase, G4-amylase, glucoamylase, glucose oxidase, glucosidase, glycolipase, hemicellulase, laccase, lipase, lysophospholipase, maltogenic amylase, mannanase, neutral protease, nuclease, oxidase, oxidoreductase, pectate lyase, pectinase, pectin esterase, pentosanase, perhydrolase, phospholipase, phytase, polygalacturonase, protease, proteinase, pullulanase, rennet enzyme, rhamnogalacturonase, subtilisin, tannase, transferase, transglutaminase, xanthanase, xylanase, xyloglucanase or mixtures thereof, particularly preferably alpha-amylase and/or protease.

6. The method according to any of claims 1 to 5, wherein the microorganism is *Bacillus pumilus* DSM 14395.

7. The method according to any of claims 1 to 6, wherein the microorganism has been genetically modified.

8. A microorganism comprising an expression construct which comprises a promoter and a nucleic acid coding for the protein;
the microorganism belonging to the species *Bacillus pumilus* and
the microorganism being sporulation-inhibited, preferably by means of a modification of the gene spoIV (yqfD), more particularly by means of a deletion of the gene spoIV (yqfD) or parts thereof and
the microorganism expressing the protein.

9. The microorganism according to claim 8, wherein the protein is secreted.

10. The microorganism according to claim 8 or 9, wherein
(a) the promoter comprises a nucleic acid sequence selected from
(i) nucleic acid sequence which is at least 80% identical to the nucleic acid sequence specified in SEQ ID NO. 1;
(ii) nucleic acid sequence which is at least 80% identical to the nucleic acid sequence specified in SEQ ID NO. 2;
(iii) nucleic acid sequence which is at least 80% identical to the nucleic acid sequence specified in SEQ ID NO. 3;
(iv) nucleic acid sequence which is at least 80% identical to the nucleic acid sequence specified in SEQ ID NO. 4;
and/or
(b) the protein is not naturally present in the microorganism,
and/or
(c) the protein is an enzyme, more particularly an acidic cellulase, alpha-amylase, alpha-acetodecarboxylase, aminopeptidase, amylase, arabanase, beta-glucanase, beta-glucosidase, beta-mannosidase, carrageenase, carbohydrase, catalase, cellobiose oxidase, cellulase, chymosin, endo-1,3-beta-glucanase, endo-1,3(4)-beta-glucanase, endo-1,4-beta-xylanase, endopeptidase, esterase, exopeptidase, G4-amylase, glucoamylase, glucose oxidase, glucosidase, glycolipase, hemicellulase, laccase, lipase, lysophospholipase, maltogenic amylase, mannanase, neutral protease, nuclease, oxidase, oxidoreductase, pectate lyase, pectinase, pectin esterase, pentosanase, perhydrolase, phospholipase, phytase, polygalacturonase, protease, proteinase, pullulanase, rennet enzyme, rhamnogalacturonase, subtilisin, tannase, transferase, transglutaminase, xanthanase, xylanase, xyloglucanase, more particularly protease or alpha-amylase, or mixtures thereof
and/or
(d) the microorganism is *Bacillus pumilus* DSM 14395,
and/or
(e) the microorganism has been genetically modified.

11. The use of a microorganism according to any of claims 8 to 10 for producing a protein, more particularly an enzyme.

## Revendications

1. Procédé de fabrication d'une protéine par un microorganisme, comprenant les étapes de procédé suivantes :
(a) l'introduction d'une construction d'expression dans un microorganisme, qui comprend un promoteur et un acide nucléique codant pour la protéine ;
(b) l'expression de la protéine dans le microorganisme,
le microorganisme appartenant à l'espèce *Bacillus pumilus* et
la sporulation du microorganisme étant inhibée, de préférence par une modification du gène spo IV (yqfD), notamment par une suppression du gène spo IV (yqfD) ou de parties de celui-ci.

2. Procédé selon la revendication 1, **caractérisé en ce que** la protéine est sécrétée.

3. Procédé selon la revendication 1 ou 2, **caractérisé en ce que** le promoteur comprend une séquence d'acides nucléiques qui est choisie parmi :
(a) une séquence d'acides nucléiques qui est au moins 80 % identique à la séquence d'acides nucléiques indiquée dans SEQ ID NO. 1 ;
(b) une séquence d'acides nucléiques qui est au moins 80 % identique à la séquence d'acides nucléiques indiquée dans SEQ ID NO. 2 ;
(c) une séquence d'acides nucléiques qui est au moins 80 % identique à la séquence d'acides nucléiques indiquée dans SEQ ID NO. 3 ;
(d) une séquence d'acides nucléiques qui est au moins 80 % identique à la séquence d'acides nucléiques indiquée dans SEQ ID NO. 4.

4. Procédé selon l'une quelconque des revendications 1 à 3, **caractérisé en ce que** la protéine n'est pas présente naturellement dans le microorganisme.

5. Procédé selon l'une quelconque des revendications 1 à 4, **caractérisé en ce que** la protéine est une enzyme, notamment une cellulase acide, une alpha-amylase, une alpha-acétodécarboxylase, une aminopeptidase, une amylase, une arabanase, une bêta-glucanase, une bêta-glucosidase, une bêta-mannosidase, une carraghénase, une carbohydrase, une catalase, une cellobiose-oxydase, une cellulase, une chymosine, une endo-1,3-bêta-glucanase, une endo-1,3(4)-bêta-glucanase, une endo-1,4-bêta-xylanase, une endopeptidase, une estérase, une exopeptidase, une G4-amylase, une glucoamylase, une glucose-oxydase, une glucosidase, une glycolipase, une hémicellulase, une laccase, une lipase, une lyspohospholipase, une amylase maltogène, une mannanase, une protéase neutre, une nucléase, une oxydase, une oxydoréductase, une pectate-lyase, une pectinase, une pectinestérase, une pentosanase, une perhydrolase, une phospholipase, une phytase, une polygalacturonase, une protéase, une protéinase, une pullulanase, une enzyme de présure, une rhamnogalacturonase, une subtilisine, une tannase, une transférase, une transglutaminase, une xanthanase, une xylanase, une xyloglucanase ou leurs mélanges, de manière particulièrement préférée une alpha-amylase et/ou une protéase.

6. Procédé selon l'une quelconque des revendications 1 à 5, **caractérisé en ce que** le microorganisme consiste en *Bacillus pumilus* DSM 14395.

7. Procédé selon l'une quelconque des revendications 1 à 6, **caractérisé en ce que** le microorganisme est modifié génétiquement.

8. Microorganisme contenant une construction d'expression, qui comprend un promoteur et un acide nucléique codant pour la protéine ;
le microorganisme appartenant à l'espèce *Bacillus pumilus* et
la sporulation du microorganisme étant inhibée, de préférence par une modification du gène spo IV (yqfD), notamment par une suppression du gène spo IV (yqfD) ou de parties de celui-ci, et
le microorganisme exprimant la protéine.

9. Microorganisme selon la revendication 8, **caractérisé en ce que** la protéine est sécrétée.

10. Microorganisme selon la revendication 8 ou 9, **caractérisé en ce que**
(a) le promoteur comprend une séquence d'acides nucléiques qui est choisie parmi :
(i) une séquence d'acides nucléiques qui est au moins 80 % identique à la séquence d'acides nucléiques indiquée dans SEQ ID NO. 1 ;
(ii) une séquence d'acides nucléiques qui est au moins 80 % identique à la séquence d'acides nucléiques indiquée dans SEQ ID NO. 2 ;
(iii) une séquence d'acides nucléiques qui est au moins 80 % identique à la séquence d'acides nucléiques indiquée dans SEQ ID NO. 3 ;
(iv) une séquence d'acides nucléiques qui est au moins 80 % identique à la séquence d'acides nucléiques indiquée dans SEQ ID NO. 4 ;
et/ou
(b) la protéine n'est pas présente naturellement dans le microorganisme,
et/ou
(c) la protéine est une enzyme, notamment une cellulase acide, une alpha-amylase, une alpha-acétodécarboxylase, une aminopeptidase, une amylase, une arabanase, une bêta-glucanase, une bêta-glucosidase, une bêta-mannosidase, une carraghénase, une carbohydrase, une catalase, une cellobiose-oxydase, une cellulase, une chymosine, une endo-1,3-bêta-glucanase, une endo-1,3(4)-bêta-glucanase, une endo-1,4-bêta-xylanase, une endopeptidase, une estérase, une exopeptidase, une G4-amylase, une glucoamylase, une glucose-oxydase, une glucosidase, une glycolipase, une hemicellulase, une laccase, une lipase, une lyspohospholipase, une amylase maltogène, une mannanase, une protéase neutre, une nucléase, une oxydase, une oxydoréductase, une pectate-lyase, une pectinase, une pectinestérase, une pentosanase, une perhydrolase, une phospholipase, une phytase, une polygalacturonase, une protéase, une protéinase, une pullulanase, une enzyme de présure, une rhamnogalacturonase, une subtilisine, une tannase, une transférase, une transglutaminase, une xanthanase, une xylanase, une xyloglucanase, notamment une protéase ou une alpha-amylase, ou leurs mélanges,
et/ou
(d) le microorganisme consiste en *Bacillus pumilus* DSM 14395,
et/ou
(e) le microorganisme est modifié génétiquement.

11. Utilisation d'un microorganisme selon l'une quelconque des revendications 8 à 10 pour la fabrication d'une protéine, notamment d'une enzyme.
